# EUROPÄISCHE PATENTSCHRIFT

(11) **EP 0 629 186 B1**
(45) Veröffentlichungstag und Bekanntmachung des Hinweises auf die Patenterteilung: **13.05.1998**
(21) Anmeldenummer: 93905290.8
(22) Anmeldetag: 03.03.1993
(51) Int. Cl.: C07C 209/60

(54) **VERFAHREN ZUR HERSTELLUNG VON TERTIÄREM BUTYLAMIN**
PROCESS FOR PREPARING TERTIARY BUTYLAMINE
PROCEDE DE PREPARATION DE BUTYLAMINE TERTIAIRE

(30) Priorität: 05.03.1992 DE 4206992
(43) Veröffentlichungstag der Anmeldung: 21.12.1994
(73) Patentinhaber: Akzo Nobel N.V., 6824 BM Arnhem (NL)
(72) Erfinder: BERGFELD, Manfred, D-8765 Erlenbach-Mechenhard (DE); NYWLT, Martin, D-8753 Obernburg (DE)
(74) Vertreter: Fett, Günter
(86) Internationale Anmeldenummer: EP9300482
(87) Internationale Veröffentlichungsnummer: WO9317995

(56) Entgegenhaltungen:
- EP-A- 0 039 918
- EP-A- 0 305 564
- DE-A- 3 326 579
- US-A- 4 307 250
- JOURNAL OF ORGANIC CHEMISTRY. Bd. 53, Nr. 19, 16. September 1988, WASHINGTON US Seiten 4594 - 4596 M. DEEBA ET AL. 'HETEROGENEOUS ACID-CATALYZED AMINATION OF ISOBUTENE TO TERT-BUTYLAMINE'
- CHEMISTRY LETTERS. Nr. 6, 1991, TOKYO JP M.TABATA ET AL 'DIRECT AMINATION OF 2-METHYLPROPENE WITH AMMONIA INTO T-BUTYLAMINE ON PROTON-EXCHANGED ZSM-5 ZEOLITE CATALYST.'
- KUNSTSTOFFTECHNISCHES WÖRTERBUCH - PLASTICS TECHNICAL DICTIONARY, A.M. Wittfoht, Bd. 1 (Englisch-Deutsch), S. 95, 356-7, Carl Hanser Verlag, München, DE, 1961

## Beschreibung

Die Erfindung betrifft ein Verfahren zur Herstellung von tertiärem Butylamin aus Ammoniak und Isobuten.

Tertiäres Butylamin ist ein technisch sehr wichtiges Zwischenprodukt für die Herstellung von Farbstoffen, Pflanzenschutzmitteln und Kautschukchemikalien.

Die industriellen Verfahren beruhen auf der sogenannten Ritter-Reaktion, bei dem durch Addition von Blausäure und Wasser an Olefine oder der Substitutionsreaktion von Alkoholen mit Blausäure und Wasser zunächst ein Formamidderivat entsteht, dessen Hydrolyse dann zum gewünschten Amin führt. Als Katalysator werden nahezu äquimolare Mengen an Schwefelsäure eingesetzt. Als Nebenprodukt fällt Ameisensäure an. Der Prozeß ist sehr stark mit toxischen und agressiven Substanzen belastet, deren Handling, Abtrennung und Aufarbeitung einen erheblichen technischen und finanziellen Aufwand erfordern.

Die der vorliegenden Erfindung zugrunde liegende Addition von Ammoniak an Isobuten ist eine Gleichgewichtsreaktion und wird seit langem untersucht, ohne jedoch zu einer technischen Lösung gelangt zu sein. Durch die Lage des Gleichgewichtes sind bei technischen Reaktionsbedingungen nur relativ geringe Umsätze von weniger als 20 % zu erreichen. Eine wirtschaftliche Rückführung der nicht-umgesetzten Edukte ist jedoch nur bei sehr hoher Selektivität zu tert. Butylamin zu erzielen. Ein Verfahren, basierend auf der Additionsreaktion, muß deshalb durch hohe Produktselektivität, gute Katalysatorstandzeit sowie eine hohe Katalysatoraktivität ausgezeichnet sein.

Es hat in der Vergangenheit nicht an Versuchen gefehlt, aus der Additionsreaktion ein technisch verwertbares Verfahren zu entwickeln, jedoch gelang es bisher nicht, ein solches Verfahren zu entwickeln, das allen Erfordernissen, wie nachfolgend kurz beschrieben, genügt.

Ein technisch verwertbares Verfahren ist vor allen Dingen dadurch gekennzeichnet, daß das Verhältnis von Recycleströmen und Produktströmen minimiert werden muß. Dies bedeutet den Einsatz äquimolarer Mengen an Ammoniak und Isobuten. Diese Maßnahme zur Optimierung des Recycleverhältnisses darf jedoch nicht die Selektivität des Katalysators zu tertiäres Butylamin verringern.

Ein solches verwertbares technisches Verfahren muß aber auch Konzentrationsschwankungen tolerieren können, das heißt, daß auch ein Unterschuß an Ammoniak nicht zu einer Erhöhung der Nebenproduktbildung (Oligomerisierung und Cracken von Isobutenen, Bildung von ditertiären Butylaminen) führen darf.

Bei den bisher bekannten Verfahren zur Herstellung von tertiärem Butylamin beträgt jedoch das Verhältnis Ammoniak : Isobuten 1,5 : 1 und mehr.

An den für die Reaktion geeigneten Katalysator werden in erster Linie im Grunde zwei widersprechende Forderungen gestellt, nämlich einmal soll der Katalysator eine hohe Aktivität aufweisen, um möglichst nahe an den Gleichgewichtsumsatz zu gelangen, auf der anderen Seite soll er jedoch die Nebenproduktbildung und Verkokung nicht fördern (hohe Selektivität).

Auch muß der Katalysator bei Temperaturen unterhalb etwa 320°C genügend reaktiv sein, da sonst die Reaktionsteilnehmer und Reaktionsprodukte thermisch zersetzt werden und erhöhte Verkokung bzw. Nebenproduktbildung eintritt.

Bei einem im technischen Maßstab brauchbaren Recycleverfahren ist die Unterdrückung von Nebenprodukten wie etwa leicht flüchtige Amine (Methylamin) sehr wichtig, da diese sonst als Leichtsieder wieder der Reaktion zugeführt werden und dort zu gemischten Aminen (etwa tert. Butylmethylamin) reagieren können. Bei mehreren Zyklen würd sich der Anteil dieser Nebenprodukte kontinuierlich anreichern, so daß zum einen keine genügend hohe Ausbeute an tert. Butylamin erzielt wird und zum anderen sich die Herstellung von reinem (> 99,5%) tert. Butylamin durch kostspielige Trennoperationen dramatisch verteuert.

Ebenso ist es für ein technisches Verfahren erforderlich, daß mit technischen Rohstoffen gearbeitet werden kann, d.h. diese ohne kostspielige Vorbehandlung eingesetzt werden können. Im allgemeinen haben daher solche Rohstoffe auch gewisse Anteile an Verunreinigungen, die dann natürlich im Recyclezyklus sich inert verhalten sollen und keinen negativen Einfluß auf die Aktivität/Selektivität und Standzeit des Katalysators besitzen und auf einfache Art und Weise abgetrennt werden können.

Insbesondere sollte n-Buten, das normalerweise im technischen Isobuten (Edukt) in geringen Mengen vorhanden ist, nicht mit Ammoniak zu Isobutylamin umgesetzt werden, da sonst die Produktspezifikationen (hochreines (> 99,5%)) tert. Butylamin) nicht erreicht werden. Auch würde in diesem Fall das geschilderte Verfahren des Recyclens durch aufwendige Trennstufen (z.B. Trennung TBA/Isobutylen) gravierend belastet werden.

Die Synthese von tert. Butylamin aus Ammoniak und Isobuten ist prinzipiell beispielsweise aus der EP-A 0 039 918 bekannt, wo bei vergleichsweise geringem Umsatz gute Selektivitäten erreicht werden können, bzw. daß bei einer Erhöhung des Umsatzes die Selektivität jedoch zurückgeht. Die dort genannten Katalysatoren besitzen wegen ihrer schnellen Verkokung nur geringe Standzeiten.

Die EP-A-0 305 564 beschreibt ein Verfahren zur Herstellung von Aminen durch Umsetzung eines Olefins mit 2 - 8 C-Atomen, insbesondere eines verzweigten C₄-Olefins, mit Ammoniak oder einem primären oder sekundären Amin unter Verwendung eines teilweise dealuminierten Aluminosilikats. Darin wird hervorgehoben, daß im Gegensatz zu den bisher bei der Aminierung von Olefinen als Katalysatoren verwendeten synthetischen kristallinen Aluminosilikaten, deren Kationen bevorzugt durch ein seltenes Erdmetall und H-Ionen ausgetauscht werden, teilweise dealuminierte Aluminosilikate eine höhere Umsatzgeschwindigkeit und längere Standzeiten bewirken, während die Selektivitäten gleich bleiben. Die höchsten Selektivitäten werden bei der Aminierung von Olefinen in dem Druckbereich von 42-77 bar erzielt und betragen bei geradkettigen Olefinen mehr als 95%, bei dem verzweigten Isobutylen jedoch nur bis zu 72% (US-PS 4,307,250).

Wird der Katalysator nach dem Stand der Technik, wie er etwa der DE-OS 33 26 579 zu entnehmen ist, auf längere Standzeiten, d.h. einen geringeren Verkokungsgrad eingestellt, so werden tert. Butylamin-Selektivitäten von weniger als 98,7% erreicht. Der in der DE-OS 39 40 349 genannte Galliumsilikat-Zeolith zeigt ebenfalls eine Selektivität von etwa nur 98 %; wobei zusätzlich für eine quantitative Umsetzung jedoch sehr hohe Drücke erforderlich sind.

Auch bei einer Reihe von anderen Verfahren, die sich aus dem Stand der Technik ergeben, entstehen aufgrund des stöchiometrischen Gleichgewichts bei der klassischen Substitutions-Reaktion Nebenprodukte, die in aufwendigen Reinigungsschritten entfernt werden müssen.

Der Erfindung liegt die Aufgabe zugrunde, ein Verfahren zur Verfügung zu stellen, mit dem sich tert. Butylamin umweltfreundlich und auf einfache Art und Weise herstellen läßt und bei dem keine Nebenprodukte entstehen, die aufwendig aus dem Reaktionsgemisch entfernt werden müssen. Außerdem soll der Katalysator eine lange Betriebsdauer haben sowie eine hohe Selektivität und Aktivität aufweisen.

Gegenstand der Erfindung ist ein Verfahren zur Herstellung von tertiärem Butylamin mit einer Reinheit von wenigstens 99,5 %, welches direkt ohne weitere Reinigung verwendet werden kann, indem man in einem Reaktor Isobuten und Ammoniak in der Gasphase über einem dealuminierten Silica-Alumina-Katalysator umsetzt, wobei der Katalysator in saurer Form vorliegt, das dadurch gekennzeichnet ist,
(a) daß der Katalysator eine Kristallinität von mindestens 95 % besitzt,
(b) der Na₂O-Gehalt des Katalysators weniger als 0,2 Gew. % beträgt,
(c) das Si/Al-Verhältnis des Katalysators 7 bis 397 ist,
(d) der Verkokungsgrad des Katalysators weniger als 1% in 200 h und weniger als 1,5% in 400 h beträgt,
(e) das molare Verhältnis von Ammoniak zu Isobuten 0,3 bis 10:1,
(f) die Reaktionstemperatur 200 bis 350°C,
(g) der Druck 100 bis 250 bar,
(h) das Verhältnis Edukte zu Katalysator 5 bis 60 mol/kg Katalysator pro Stunde beträgt und
(i) die nicht umgesetzten Edukte von dem gebildeten tertiären Butylamin abgetrennt werden.

Bei Einsatz eines technisches Isobutens (Reinheit < 99,5%) beträgt die Reinheit des gebildeten tert. Buylamins nach gaschromatographischer Analyse mehr als 99,5%.

Bei Einsatz eines reinen Isobutens (Reinheit > 99,9%) beträgt die Reinheit des gebildeten tert. Butylamins nach gaschromatographischer Analyse mehr als 99,8%.

Die Reinheit wurde gaschromatographisch nach üblichen Methoden bestimmt (Ofentemperatur: 80°C; N₂ als Trägergas; FID-Detektor; Probenschleife: 12,5 µl; 30-m-Säule).

Bevorzugt wird die Reaktion in einem Rohrreaktor ausgeführt.

In einer anderen Ausführungsform wird die Reaktion in einem adiabatischen Schüttschichtreaktor ausgeführt.

Der Werkstoff des Reaktors ist bevorzugt gegenüber den Reaktionsteilnehmern inert.

In einer Ausführungsform der Erfindung ist der Reaktorwerkstoff ein Cr-Ni-Stahl.

In einer anderen Ausführungsform ist der Reaktorwerkstoff ein nickelbasierter Werkstoff vom Typ Inconel 600.

Besonders bevorzugt beträgt die Katalysatorbelastung 10 bis 30 mol/(kg.Kat.h).

Das Anfahren des Reaktors geschieht durch folgende Maßnahmen:
a) Mehrfaches Spülen mit N₂ zur vollständigen Entfernung der Luft,
b) Aufheizen auf Betriebstemperatur,
c) Spülen mit Ammoniak,
d) den Betriebsdruck unter Ammoniakatmosphäre einstellen,
e) Isobuten bis zu dem gewünschten Molverhältnis in den Reaktor eindosieren,
f) nach Einstellung des stationären Reaktionsgleichgewichts zudosieren der Edukte im gewünschten Molverhält-nis und entsprechend kontinuierlicher Abzug des Reaktionsgemisches.

Bevorzugt beträgt das molare Verhältnis von Ammoniak zu Isobuten 0,9 : 1 bis 4 : 1.

Das Verfahren ist weiterhin dadurch gekennzeichnet, daß auch bei molaren Verhältnissen von NH₃ zu Isobuten von weniger als 1 : 1 die Selektivität des Katalysators > 99,8% ist.

In einer Ausführungsform der Erfindung handelt es sich bei dem Katalysator um einen Zeolithen vom Pentasiltyp.

In einer anderen Ausführungsform der Erfindung handelt es sich bei dem Katalysator um einen Zeolithen vom Y-Typ.

Das Si/Al-Verhältnis des Katalysators wird durch Dealuminierung eingestellt.

Bevorzugt wird die Dealuminierung mit Wasserdampf durchgeführt.

In einer anderen Ausführungsform der Erfindung wird die Dealuminierung mit SiCl₄ durchgeführt.

In einer weiteren Ausführungsform wird die Dealuminierung mit Mineralsäure durchgeführt.

Die Dealuminierung kann auch mit organischen Komplexbildnern z.B. EDTA durchgeführt werden.

Die Dealuminierung kann auch mit Hexafluorosilikaten durchgeführt werden.

Bevorzugt erfolgt die Dealuminierung mittels ein- oder mehrfacher Wasserdampfbehandlung.

Bevorzugt wird die acide Form des Katalysators Ionenaustausch mit Brönstedsäuren gebildet.

Die acide Form kann auch durch Ionenaustausch mit Ammoniumchlorid gebildet werden.

Besonders bevorzugt enthält der Katalysator weniger als 0,05% Na₂O.

Bevorzugt wird der Katalysator durch Extrudieren oder Granulieren mit Silica oder Alumina in eine technisch handhabbare Form gebracht.

Bevorzugt wird der Katalysator im Verhältnis 80 : 20 (Zeolith : Alumina) extrudiert.

Die Extrudate weisen eine Länge von 4 bis 20 mm und einen Durchmesser von 1,5 bis 4 mm auf.

Bevorzugt weist der Katalysator eine Standzeit von mindestens 200 h auf.

Besonders bevorzugt beträgt die Standzeit mindestens 400 h.

Bevorzugt erfolgt die Abtrennung der nicht umgesetzten Edukte von dem gebildeten tertiären Butylamin mittels Destillation.

Bei der Destillation werden Ammoniak und Isobuten als Azeotrop abgezogen.

Die Destillation erfolgt bei einem Druck von 1 bis 30 bar, bevorzugt jedoch bei 30 bar.

Bevorzugt wird die Destillation bei 30 bar in einer Hauptkolonne mit weniger als 60 Stufen durchgeführt.

Besonders bevorzugt weist die Hauptkolonne 10 bis 30 Stufen auf.

Bevorzugt weist die Hauptkolonne ein Rücklaufverhältnis von 0,5 bis 10, besonders bevorzugt von 0,6 bis 3 auf.

Bevorzugt wird das Verfahren so ausgeführt, daß rezirkulierendes Ammoniak und Isobuten in flüssigem Zustand auf den Reaktionsdruck komprimiert werden.

Bevorzugt werden dann die Flüssigkeiten auf die erforderliche Reaktionstemperatur erhitzt.

Bevorzugt wird ein geringer Teilstrom abgezweigt und von Inertanteilen (z.B. Isobutan) gereinigt wird, bevor dieser in den Prozeß zurückgeführt wird.

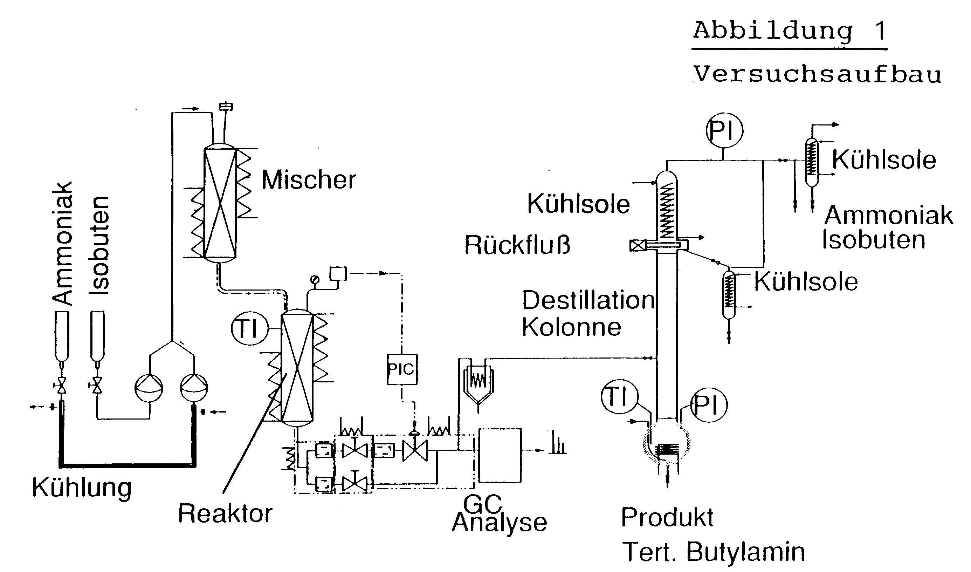

Abbildung 1 gibt eine schematische Übersicht der Versuchsdurchführung wieder.

Die Erfindung wird durch die nachstehenden Beispiele näher erläutert.

### Beispiele 1 bis 16:

Hierfür wurden drei verschiedene Katalysatorentypen hergestellt.

### Katalysator A

Ein Y-Zeolith wurde entsprechend üblicher Synthesemethoden hergestellt und mit Säure gewaschen. Nach der Extrusion mit 20 % Alumina wurde der Ionenaustausch durch zweimaliges Behandeln bei 80 °C mit NH₄Cl vervollständigt. Anschließend wurde getrocknet und bei 500 °C für 2 h calciniert. Die charakteristischen Daten des Katalysators sind:
Si/Al-Verhältnis: 13,4
Kristallinität: > 95 %
Na₂O-Gehalt: < 0,2 %

### Katalysator B

Ein HZSM-5-Zeolith wurde entsprechend üblicher Synthesemethoden hergestellt und mit Säure gewaschen. Nach der Extrusion mit 20 % Alumina wurde der Ionenaustausch durch zweimaliges Behandeln bei 80 °C mit NH₄Cl vervollständigt. Anschließend wurde getrocknet und bei 500 °C für 2 h calciniert. Die charakteristischen Daten des Katalysators sind:
Si/Al-Verhältnis: > 150
Kristallinität: > 95 %
Na₂O-Gehalt: < 0,2 %

Ein Ionenaustausch mit Kupfer wurde nach Bedarf anschließend auf konventionellem Wege durchgeführt. In den Beispielen 8 bis 10 wurde ein mit Kupfer dotierter ZSM-5-Zeolith eingesetzt, in den Beispielen 11 bis 13 wurde ein ZSM-Zeolith in der H-Form verwendet.

### Katalysator C

Ein Zeolith wurde entsprechend üblicher Synthesemethoden hergestellt und mit Säure gewaschen. Nach der Extrusion mit 20 % Alumina wurde der Ionenaustausch durch zweimaliges Behandeln bei 80 °C mit NH₄Cl vervollständigt. Anschließend wurde getrocknet und bei 500 °C für 2 h calciniert. Die charakteristischen Daten des Katalysators sind:
Si/Al-Verhältnis: 397
Kristallinität: > 95 %
Na₂O-Gehalt: < 0,2 %

### Reaktionsdurchführung

Alle Reaktionen wurden in einem Rohrreaktor gemäß Zeichnung (Werkstoff Inconel 600, 200 ml Volumen, Innendurchmesser 2 cm) durchgeführt. Die Edukte wurden in einer gleichartigen, mit Drahtwendeln gefüllten Mischkammer vermischt, das Produkt in einer mit Raschigringen gefüllten Destillationskolonne von den nicht-umgesetzten Edukten abgetrennt. Der Durchsatz betrug etwa 6 bis 10 mol/(kg Kat h).

**Tabelle 1**

| Beispiel | Temperatur °C | Druck bar | Katalysator | Mol-Verhältnis NH₃/Isobuten | Umsatz % |
|---|---|---|---|---|---|
| 1 | 300 | 100 | A | 2 : 1 | 6,4 |
| 2 | 300 | 100 | A | 4 : 1 | 6,5 |
| 3 | 250 | 100 | A | 2 : 1 | 2,2 |
| 4 | 280 | 100 | A | 2 : 1 | 5,5 |
| 5 | 300 | 100 | C | 2 : 1 | 6,3 |
| 6 | 300 | 100 | C | 1 : 1 | 5,6 |
| 7 | 300 | 100 | C | 0,9 : 1 | 5,4 |
| 8 | 300 | 100 | B | 2 : 1 | 7,1 |
| 9 | 280 | 100 | B | 2 : 1 | 6,5 |
| 10 | 280 | 100 | B | 1 : 1 | 6,4 |
| 11 | 300 | 150 | B | 2 : 1 | 9,5 |
| 12 | 300 | 200 | B | 2 : 1 | 10,2 |
| 13 | 300 | 250 | B | 2 : 1 | 11,6 |
| 14 | 300 | 100 | C | 0,3 : 1 | 3,3 |
| 15 | 300 | 100 | C | 0,5 : 1 | 2,2 |
| 16 | 300 | 100 | C | 0,3 : 1 | 1,8 |

### Beispiele 17 bis 22:

Für die Beispiele 17 bis 12 wurde der Katalysator A verwendet.

Die Tabelle 2 gibt die Ergebnisse dieser Versuche wieder.

**Tabelle 2**

| Beispiel | Temperatur °C | Druck bar | Ausbeute TBA (Gew.%) | Nebenprodukte (Fl.%, nach GC) |
|---|---|---|---|---|
| 17 | 250 | 100 | 3,15 | keine detektiert |
| 18 | 280 | 100 | 6,95 | keine detektiert |
| 19 | 300 | 100 | 6,6 | keine detektiert |
| 20 | 300 | 150 | 7,75 | keine detektiert |
| 21 | 300 | 200 | 11,95 | 0,8 bis 1,5 |
| 22 | 350 | 200 | 7,23 | 0,6 bis 3,5 |

Die Molverhältnisse NH₃: Isobuten lagen zwischen 1,8 und 2,2 : 1.

Bei allen Versuchen, die mit einer Temperatur von 300°C und einem Druck von weniger als 200 bar durchgeführt wurden, ist die Selektivität unabhängig vom Mol-Verhältnis Ammoniak/ Isobuten und beträgt gemäß der zuvor definierten gaschromatographischen Methode 100%.

## Patentansprüche

1. Verfahren zur Herstellung von tertiärem Butylamin mit einer Reinheit von wenigstens 99,5 %, welches direkt ohne weitere Reinigung verwendet werden kann, indem man in einem Reaktor Isobuten und Ammoniak in der Gasphase über einem dealuminierten Silica-Alumina-Katalysator umsetzt, wobei der Katalysator in saurer Form vorliegt, dadurch gekennzeichnet,
(a) daß der Katalysator eine Kristallinität von mindestens 95 % besitzt,
(b) der Na₂O-Gehalt des Katalysators weniger als 0,2 Gew. % beträgt,
(c) das Si/Al-Verhältnis des Katalysators 7 bis 397 ist,
(d) der Verkokungsgrad des Katalysators weniger als 1 % in 200 h und weniger als 1,5% in 400 h beträgt,
(e) das molare Verhältnis von Ammoniak zu Isobuten 0,3 bis 10 : 1,
(f) die Reaktionstemperatur 200 bis 350° C,
(g) der Druck 100 bis 250 bar,
(h) das Verhältnis Edukte zu Katalysator 5 bis 60 mol/kg Katalysator pro Stunde beträgt und
(i) die nicht umgesetzten Edukte von dem gebildeten tertiären Butylamin abgetrennt werden.

2. Verfahren nach Anspruch 1, dadurch gekennzeichnet, daß technisches Isobuten (Reinheit < 99,5%) eingesetzt wird.

3. Verfahren nach Anspruch 1, dadurch gekennzeichnet, daß reines Isobuten (Reinheit > 99,9%) eingesetzt wird.

4. Verfahren nach einem oder mehreren der Ansprüche 1 bis 3, dadurch gekennzeichnet, daß das Verhältnis Edukte zu Katalysator 10 bis 30 mol/kg Kat.h beträgt.

5. Verfahren nach einem oder mehreren der Ansprüche 1 bis 4, dadurch gekennzeichnet, daß das molare Verhältnis von Ammoniak zu Isobuten 0,9:1 bis 4:1 beträgt.

6. Verfahren nach einem oder mehreren der Ansprüche 1 bis 5, dadurch gekennzeichnet, daß der Katalysator weniger als 0,05% Na₂O enthält.

7. Verfahren nach einem oder mehreren der Ansprüche 1 bis 6 dadurch gekennzeichnet, daß die Abtrennung der Edukte mittels Destillation erfolgt.

8. Verfahren nach Anspruch 7, dadurch gekennzeichnet, daß bei der Destillation Ammoniak und Isobuten als Azeotrop abgezogen werden.

9. Verfahren nach Anspruch 7, dadurch gekennzeichnet, daß die Destillation bei einem Druck von 1 bis 30 bar, bevorzugt jedoch bei 30 bar erfolgt.

10. Verfahren nach einem oder mehreren der Ansprüche 1 bis 9, dadurch gekennzeichnet, daß die nicht umgesetzten Produkte in flüssiger Form in die Reaktion zurückgeführt werden.

11. Verfahren nach Anspruch 10, dadurch gekennzeichnet, daß das rezirkulierende Ammoniak und Isobuten in flüssigem Zustand auf den Reaktionsdruck komprimiert und auf die erforderliche Reaktionstemperatur erhitzt werden.

12. Verfahren nach den Ansprüchen 10 bis 11, dadurch gekennzeichnet, daß ein geringer Teilstrom abgezweigt und von Inertanteilen (z.B. Isobutan) gereinigt wird, bevor dieser in den Prozeß zurückgeführt wird.

13. Verfahren nach einem oder mehreren der Ansprüche 1 bis 6, dadurch gekennzeichnet, daß das Anfahren des Reaktors durch folgende Maßnahmen geschieht:
a) Mehrfaches Spülen mit N₂ zur vollständigen Entfernung der Luft,
b) Aufheizen auf Betriebstemperatur,
c) Spülen mit Ammoniak,
d) den Betriebsdruck unter Ammoniakatmosphäre einstellen,
e) Isobuten bis zu dem gewünschten Molverhältnis in den Reaktor eindosieren,
f) nach Einstellung des stationären Reaktionsgleichgewichts Zudosieren der Edukte im gewünschten Molverhältnis und entsprechend kontinuierlicher Abzug des Reaktionsgemisches.

## Claims

1. A process for preparing tertiary butylamine having a purity of at least 99.5%, which can be used directly without further purification, by reacting isobutene and ammonia in the gas phase in a reactor over a dealuminised silica-alumina catalyst, the catalyst being present in acid form,
characterised in that
a) the catalyst has a crystallinity of at least 95%,
b) the Na₂O content of the catalyst is less than 0.2 wt.%,
c) the Si/Al ratio of the catalyst is from 7 up to 397,
d) the degree of carbonisation of the catalyst is less than 1% in 200 hours and less than 1.5% in 400 hours,
e) the molar ratio of ammonia to isobutene is from 0.3 up to 10:1,
f) the reaction temperature is 200°C to 350°C,
g) the pressure is 100 bar to 250 bar,
h) the ratio of educts to catalyst is from 5 up to 60 mol/kg catalyst per hour and
i) the unreacted educts are separated from the tertiary butylamine formed.

2. A process according to claim 1, characterised in that technical isobutene (purity < 99.5%) is used.

3. A process according to claim 1, characterised in that pure isobutene (purity > 99.9%) is used.

4. A process according to one or more of claims 1 to 3, characterised in that the ratio of educts to catalyst is from 10 to 30 mol/kg cat. h.

5. A process according to one or more of claims 1 to 4, characterised in that the molar ratio of ammonia to isobutene is from 0.9:1 to 4:1.

6. A process according to one or more of claims 1 to 5, characterised in that the catalyst contains less than 0.05% Na₂O.

7. A process according to one or more of claims 1 to 6, characterised in that the separation of the educts is carried out by means of distillation.

8. A process according to claim 7, characterised in that ammonia and isobutene are removed as an azeotrope during the distillation.

9. A process according to claim 7, characterised in that the distillation is carried out at a pressure of from 1 bar to 30 bar, but preferably at 30 bar.

10. A process according to one or more of claims 1 to 9, characterised in that the unreacted products are returned in liquid form to the reaction.

11. A process according to claim 10, characterised in that the recirculating ammonia and isobutene in the liquid state are compressed to the reaction pressure and are heated to the required reaction temperature.

12. A process according to claims 10 to 11, characterised in that a small split stream is diverted and is purified of inert constituents (for example, isobutane), before being returned to the process.

13. A process according to one or more of claims 1 to 6, characterised in that the reactor is started up by means of the following steps:
a) repeated flushing with N₂ for complete removal of the air,
b) heating up to the operating temperature,
c) flushing with ammonia,
d) adjustment of the operating pressure under an atmosphere of ammonia,
e) metering of isobutene into the reactor up to the required molar ratio,
f) after adjustment of the stationary reaction equilibrium, charging of the educts in the required molar ratio and corresponding continuous withdrawal of the reaction mixture.

## Revendications

1. Procédé de préparation de *tert*-butylamine pure à au moins 99,5 % utilisable directement sans purification supplémentaire, dans lequel on fait réagir dans un réacteur de l'isobutène et de l'ammoniac en phase gazeuse au dessus d'un catalyseur de type aluminosilicate désaluminisé, le catalyseur étant présent sous forme acide, lequel procédé est caractérisé en ce que
(a) le catalyseur a un taux de cristallinité d'au moins 95 %,
(b) la teneur en Na₂O du catalyseur est inférieure à 0,2 % en poids,
(c) le rapport Si/Al du catalyseur va de 7 à 397,
(d) le taux de cokage du catalyseur est inférieur à 1 % au bout de 200 h et inférieur à 1,5 % au bout de 400 h,
(e) le rapport molaire ammoniac/isobutène va de 0,3 à 10/1,
(f) la température réactionnelle est de 200 à 350 °C,
(g) la pression est de 100 à 250 bars,
(h) le rapport des réactifs de départ au catalyseur est de 5 à 60 moles par kg de catalyseur et par heure et
(i) les réactifs de départ n'ayant pas réagi sont séparés de la *tert*-butylamine formée.

2. Procédé conforme à la revendication 1, caractérisé en ce que l'on utilise de l'isobutène de qualité technique (pureté inférieure à 99,5 %).

3. Procédé conforme à la revendication 1, caractérisé en ce que l'on utilise de l'isobutène pur (pureté supérieure à 99,9 %).

4. Procédé conforme à une ou plusieurs des revendications 1 à 3, caractérisé en ce que le rapport des réactifs de départ au catalyseur va de 10 à 30 moles par kg de catalyseur et par heure.

5. Procédé conforme à une ou plusieurs des revendications 1 à 4, caractérisé en ce que le rapport molaire d'ammoniac à isobutène est compris dans l'intervalle allant de 0,9 : 1 à 4 : 1.

6. Procédé conforme à une ou plusieurs des revendications 1 à 5, caractérisé en ce que le catalyseur contient moins de 0,05 % de Na₂O.

7. Procédé conforme à une ou plusieurs des revendications 1 à 6, caractérisé en ce que la séparation des réactifs de départ se fait par distillation.

8. Procédé conforme à la revendication 7, caractérisé en ce que la distillation élimine de l'ammoniac et de l'isobutène sous forme d'un azéotrope.

9. Procédé conforme à la revendication 7, caractérisé en ce que la distillation se fait à une pression comprise dans l'intervalle allant de 1 à 30 bars, mais de préférence égale à 30 bars.

10. Procédé conforme à une ou plusieurs des revendications 1 à 9, caractérisé en ce que les réactifs de départ n'ayant pas réagi sont réintroduits dans le mélange réactionnel sous forme liquide.

11. Procédé conforme à la revendication 10, caractérisé en ce que l'ammoniac et l'isobutène recyclés sont comprimés à l'état liquide jusqu'à la pression réactionnelle puis chauffés à la température réactionnelle requise.

12. Procédé conforme aux revendications 10 à 11, caractérisé en ce que l'on dérivé un flux partiel faible pour le débarrasser de ses composantes inertes (par exemple d'isobutane), avant de le réintroduire dans le procédé.

13. Procédé conforme à une ou plusieurs des revendications 1 à 6, caractérisé en ce que le démarrage du réacteur implique les opérations suivantes :
(a) purge répétée avec du N₂ afin de chasser parfaitement l'air présent,
(b) chauffage à la température de fonctionnement,
(c) purge avec de l'ammoniac,
(d) ajustement de la pression de fonctionnement sous atmosphère d'ammoniac,
(e) introduction dans le réacteur d'une quantité déterminée d'isobutène jusqu'à obtention du rapport molaire désiré,
(f) après établissement de l'équilibre réactionnel stationnaire, addition des réactifs de départ avec un rapport molaire souhaité et soutirage continu correspondant du mélange réactionnel.
